(19) Europäisches Patentamt
European Patent Office
Office européen des brevets

(11) Veröffentlichungsnummer: **0 555 770 A1**

(12) **EUROPÄISCHE PATENTANMELDUNG**

(21) Anmeldenummer: **93101723.0**

(22) Anmeldetag: **04.02.93**

(51) Int. Cl.5: **C07D 401/12**, C07D 491/04, C07D 213/75, A01N 47/36, //(C07D401/12,239:00,213:00), (C07D401/12,251:00,213:00), (C07D401/12,249:00,213:00), (C07D491/04,307:00,239:00), (C07D491/04,311:00,239:00)

(30) Priorität: **14.02.92 DE 4204411**

(43) Veröffentlichungstag der Anmeldung:
**18.08.93 Patentblatt 93/33**

(84) Benannte Vertragsstaaten:
**AT CH DE ES FR GB IT LI NL PT SE**

(71) Anmelder: **HOECHST AKTIENGESELLSCHAFT**

**D-65926 Frankfurt(DE)**

(72) Erfinder: **Schutze, Rainer, Dr.**
**Pfahlgrabenstrasse 50**
**W-6270 Idstein/Ts(DE)**
Erfinder: **Kehne, Heinz, Dr.**
**Iltisweg 7a**
**W-6238 Hofheim am Taunus(DE)**
Erfinder: **Bauer, Klaus, Dr.**
**Doorner Strasse 53d**
**W-6450 Hanau(DE)**
Erfinder: **Bieringer, Hermann, Dr.**
**Eichenweg 26**
**W-6239 Eppstein/Ts(DE)**

(54) **N-Heteroaryl-N'-(pyrid-2-yl-sulfonyl)-harnstoffe, Verfahren zu ihrer Herstellung und ihre Verwendung als Herbizide und Pflanzenwachstumsregulatoren.**

(57) Verbindungen der Formel (I)

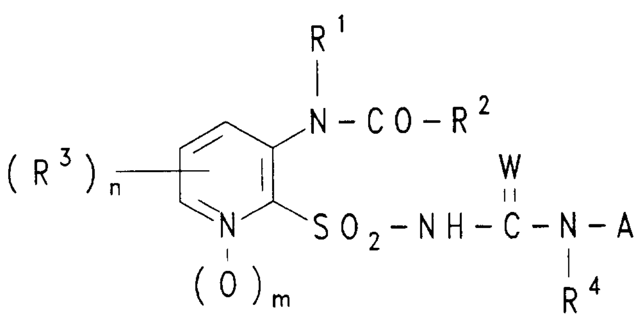

(I)

worin
$R^1$-$R^4$, A, n, m und w wie in Anspruch 1 definiert sind, eignen sich als Herbizide zur Bekämpfung von Schadpflanzen und lassen sich unter anderem aus den Sulfonamiden der Formel (II),

EP 0 555 770 A1

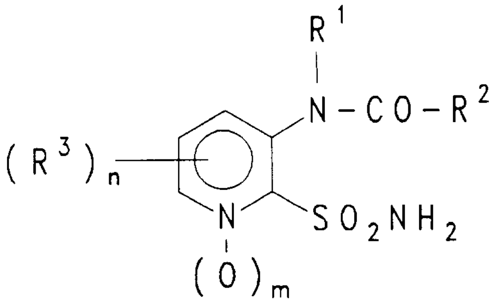

(II)

durch Umsetzung mit Carbamaten R*O-CO-NR⁴-A herstellen.

Die Erfindung betrifft das technische Gebiet der Herbizide und Pflanzenwachstumsregulatoren, insbesondere der Herbizide zur selektiven Bekämpfung von Unkräutern und Ungräsern in Nutzpflanzenkulturen.

Es ist bekannt, daß einige 2-Pyridylsulfonylharnstoffe herbizide und pflanzenwachstumsregulierende Eigenschaften besitzen; vergleiche EP-A-13 480, EP-A-272 855, EP-A-84224, US-A-4421550, EP-A-103543 (US-A-4 579 583) US-A-4487626, EP-A-125864, WO 88/04297 und WO 91/10660 (ZA 91/0173).

Es wurden nun weitere 2-Pyridylsulfonylharnstoffe mit speziellen Resten in 3-Position des Pyridylrestes gefunden, die als Herbizide und Pflanzenwachstumsregulatoren geeignet sind.

Gegenstand der vorliegenden Erfindung sind Verbindungen der Formel (I) oder deren Salze,

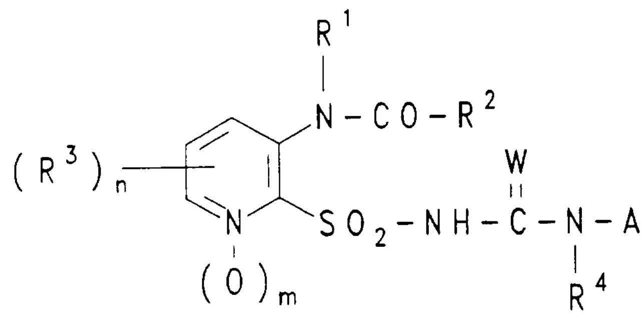

(I)

worin

| | |
|---|---|
| $R^1$ | H, $(C_1$-$C_6)$Alkyl, das unsubstituiert oder durch einen oder mehrere Reste aus der Gruppe Halogen, Nitro, $(C_1$-$C_4)$Alkoxy, $(C_3$-$C_6)$Cycloalkyl, Aryl und substituiertes Aryl substituiert ist, oder Aryl, wobei der Arylrest unsubstituiert oder durch einen oder mehrere Reste aus der Gruppe Halogen, Nitro, $(C_1$-$C_4)$Alkyl, $(C_1$-$C_4)$Haloalkyl und $(C_1$-$C_4)$Alkoxy substituiert ist, |
| $R^2$ | H, $(C_1$-$C_6)$Alkyl, $(C_2$-$C_6)$Alkenyl oder $(C_2$-$C_6]$Alkinyl, wobei jeder der drei letztgenannten Reste unsubstituiert oder durch einen oder mehrere Reste aus der Gruppe Halogen, $(C_1$-$C_4)$Alkoxy, $(C_1$-$C_4)$Alkylthio, $(C_1$-$C_4)$-Alkylsulfinyl, $(C_1$-$C_4)$-Alkylsulfonyl, Nitro, Cyano und Thiocyanato substituiert ist, oder $(C_1$-$C_6)$Alkoxy oder $(C_1$-$C_6)$Alkylthio, wobei die letztgenannten 2 Reste unsubstituiert oder durch einen oder mehrere Reste aus der Gruppe Halogen, $(C_1$-$C_4)$Alkoxy, $(C_1$-$C_4)$Alkylthio, Aryl und substituiertes Aryl substituiert sind, $(C_3$-$C_7)$Cycloalkyl oder $(C_3$-$C_7)$Cycloalkoxy, wobei die letztgenannten 2 Reste unsubstituiert oder durch einen oder mehrere Reste aus der Gruppe $(C_1$-$C_4)$Alkyl, $(C_1$-$C_4)$Alkoxy, $(C_1$-$C_4)$Alkylthio, $(C_1$-$C_4)$-Haloalkyl und Halogen substituiert sind, oder Aryl, substituiertes Aryl oder einen Rest der Formel $NR^aR^b$, |
| $R^3$ | $(C_1$-$C_4)$Alkyl, $(C_1$-$C_3)$Haloalkyl, Halogen, $NO_2$, CN, $(C_1$-$C_3)$Alkoxy, $(C_1$-$C_3)$-Haloalkoxy, $(C_1$-$C_3)$Alkylthio, $(C_1$-$C_3)$Alkoxy-$(C_1$-$C_3)$alkyl, $[(C_1$-$C_3)$Alkoxy]-carbonyl, $(C_1$-$C_3)$Alkylamino, Di$[(C_1$-$C_3)$alkyl]-amino, $(C_1$-$C_6)$-Alkylsulfinyl, $(C_1$-$C_3)$Alkylsulfonyl, $SO_2HR^cR^d$ oder $C(O)NR^eR^f$, |
| $R^a$, $R^b$, $R^c$, $R^d$, $R^e$ und $R^f$ | unabhängig voneinander H, $(C_1$-$C_4)$Alkyl, $(C_3$-$C_6)$Alkenyl,$(C_3$-$C_6]$Alkinyl, [-$(C_1$-$C_4)$Alkyl]-carbonyl, Arylcarbonyl, das im Arylrest unsubstituiert oder substituiert ist, oder paarweise $R^a$ und $R^b$, $R^c$ und $R^d$ bzw. $R^e$ und $R^f$ gemeinsam mit dem sie verbindenden N-Atom einen heterocyclischen gesättigten oder ungesättigten Ring mit 3 bis 7 Ringatomen und 0, 1 oder 2 weiteren Heteroatomen aus der Gruppe N, O und S, der unsubstituiert oder substituiert ist, |
| $R^4$ | H oder $(C_1$-$C_4)$Alkyl, vorzugsweise H oder $CH_3$, insbesondere H, |
| m | 0 oder 1, vorzugsweise 0, |
| n | 0, 1 oder 2, vorzugsweise 0 oder 1, |
| A | einen Rest der Formel |

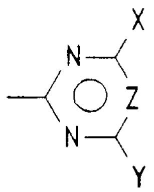 , 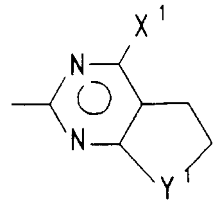 , 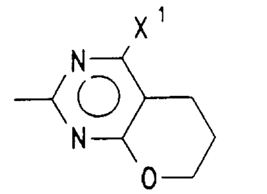 ,

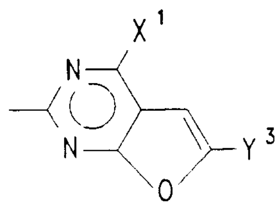 , 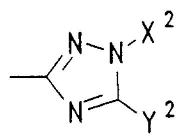 , 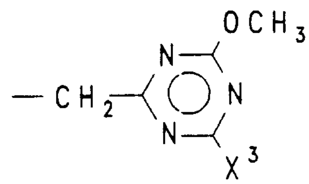 ,

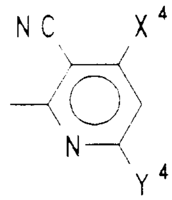 oder 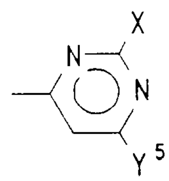

| | |
|---|---|
| X,Y | unabhängig voneinander H, Halogen, $(C_1\text{-}C_3)$Alkyl, $(C_1\text{-}C_3)$Alkoxy oder $(C_1\text{-}C_3)$Alkylthio, wobei die vorgenannten alkylhaltigen Reste unsubstituiert oder ein- oder mehrfach durch Halogen oder ein-oder zweifach durch $(C_1\text{-}C_3)$Alkoxy oder $(C_1\text{-}C_3)$Alkylthio substituiert sind, oder einen Rest der Formel $NR^5R^6$, $(C_3\text{-}C_6)$-Cycloalkyl, $(C_2\text{-}C_4)$Alkenyl, $(C_2\text{-}C_4)$Alkinyl, $(C_3\text{-}C_4)$-Alkenyloxy oder $(C_3\text{-}C_4)$Alkinyloxy, |
| $R^5$,$R^6$ | unabhängig voneinander H, $(C_1\text{-}C_3)$Alkyl oder $(C_3\text{-}C_4)$Alkenyl, |
| W | O oder S, vorzugsweise O, |
| Z | CH oder N, vorzugsweise CH, |
| $X^1$ | $CH_3$, $OCH_3$, $OC_2H_5$ oder $OCF_2H$, |
| $Y^1$ | -O- oder $-CH_2-$, |
| $X^2$ | $CH_3$, $C_2H_5$ oder $CH_2CF_3$, |
| $Y^2$ | $OCH_3$, $OC_2H_5$, $SCH_3$, $SC_2H_5$, $CH_3$ oder $C_2H_5$, |
| $X^3$ | $CH_3$ oder $OCH_3$, |
| $Y^3$ | H oder $CH_3$, |
| $X^4$ | $CH_3$, $OCH_3$, $OC_2H_5$, $CH_2OCH_3$ oder Cl, |
| $Y^4$ | $CH_3$, $OCH_3$, $OC_2H_5$ oder Cl, |
| $Y^5$ | $CH_3$, $C_2H_5$, $OCH_3$ oder Cl |

bedeuten.

In der Formel (I) und im folgenden können kohlenwasserstoffhaltige Reste wie z.B. Alkyl-, Alkoxy-, Haloalkyl- und Alkylthioreste sowie die entsprechenden ungesättigten und/oder substituierten Reste im Kohlenwasserstoffteil jeweils geradkettig oder verzweigt sein. Alkylreste, auch in den zusammengesetzten Bedeutungen wie Alkoxy, Haloalkyl usw., bedeuten Methyl, Ethyl, n- oder i-Propyl, n-, i-, t- oder 2-Butyl; Alkenyl- und Alkinylreste haben die Bedeutung der den Alkylresten entsprechenden möglichen ungesättigten Reste, wie 2-Propenyl, 2- oder 3-Butenyl, 2-Propinyl, 2- oder 3-Butinyl. Halogen bedeutet, Fluor, Chlor, Brom oder Jod. Haloalkyl bedeutet Alkyl, das durch ein oder mehrere Atome aus der Gruppe Halogen substituiert ist; Haloalkyl ist beispielsweise $CF_3$, $CHF_2$, $CH_2CF_3$. Aryl ist beispielsweise Phenyl, Naphthyl, Tetrahydronaphthyl, Indanyl, Fluorenyl und ähnliches, vorzugsweise Phenyl. Substituiertes Aryl oder substituiertes Phenyl bedeutet vorzugsweise Aryl bzw. Phenyl, das durch einen oder mehrere, vorzugsweise 1 bis 3 Reste aus der Gruppe Halogen, Alkyl, Haloalkyl, Haloalkoxy, Nitro, Cyano, Alkoxycarbonyl, Alkanoyl, Carbamoyl, Mono- und Di-alkylaminocarbonyl, Mono- und Dialkylamino, Alkylsulfinyl oder Alkylsulfonyl substituiert ist, wobei bei den alkylhaltigen Resten solche mit 1 bis 4 C-Atomen, insbesondere 1 bis 2 C-Atomen bevorzugt sind; besonders bevorzugt sind dabei Methyl, Methoxy und Chlor. Beispiele für heterocyclische Reste $R^3$ = $NR^aR^b$ sind Pyrrol, Imidazol, Pyrazol, Triazol, Pyrazolone, Oxazole, Oxazolone, Propansultame, Butansultame, Pyrrolidon, Piperidin und Morpholin.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-Isomere sind jedoch alle von den Formeln I umfaßt und können nach üblichen Methoden aus Gemischen der Stereoisomeren enthalten werden oder auch durch stereoselektive Reaktionen in Kombination mit dem Einsatz von stereochemisch reinen Ausgangsstoffen hergestellt werden.

Die Verbindungen der Formel (I) können Salze bilden, bei denen der Wasserstoff der $-SO_2$-NH-Gruppe durch ein für die Landwirtschaft geeignetes Kation ersetzt wird. Diese Salze sind beispielsweise Metall-, insbesondere Alkali- oder Erdalkalisalze, oder auch Ammoniumsalze oder Salze mit organischen Aminen. Ebenso kann Salzbildung durch Anlagerung einer starken Säure an den Pyridinteil der Verbindung der Formel (I) erfolgen. Geeignete Säuren hierfür sind starke anorganische und organische Säuren, beispielsweise HCl, HBr, $H_2SO_4$ oder $HNO_3$.

Bevorzugt sind erfindungsgemäße Verbindungen der Formel I oder deren Salze, in denen

$R^1$ H, $(C_1-C_4)$Alkyl, das unsubstituiert oder durch 1 bis 3 Reste aus der Gruppe Halogen oder durch $(C_1-C_2)$Alkoxy oder $(C_3-C_5)$Cycloalkyl substituiert ist, bedeutet.

Weiterhin sind erfindungsgemäße Verbindungen der Formel (I) oder deren Salze bevorzugt, in denen

$R^2$ H, $(C_1-C_4)$Alkyl, das unsubstituiert oder durch ein oder mehrere Halogenatome oder durch $(C_1-C_2)$Alkoxy substituiert ist, oder $(C_2-C_4)$Alkenyl, $(C_2-C_3)$Alkinyl, $(C_3-C_5)$Cycloalkyl, $(C_1-C_4)$Alkoxy, das unsubstituiert oder durch ein oder mehrere Halogenatome oder durch Phenyl substituiert ist, oder einen Rest der Formel $NR^aR^b$, worin $R^a$ und $R^b$ unabhängig voneinander H oder $(C_1-C_4)$Alkyl sind, bedeutet.

Weiter bevorzugt sind erfindungsgemäße Verbindungen der Formel (I) oder deren Salze, worin

$R^3$ $(C_1-C_4)$Alkyl, $(C_1-C_3)$Haloalkyl, Halogen, $(C_1-C_3)$Alkoxy oder Nitro und

n 0, 1 oder 2, vorzugsweise 0 oder 1, bedeuten.

Weiter bevorzugt sind Verbindungen der Formel (I) oder deren Salze, worin

$R^1$ H, $(C_1-C_4)$Alkyl, $(C_1-C_4)$Haloalkyl oder $[(C_1-C_2)$Alkoxy$]-(C_1-C_2)$alkyl,

$R^2$ H, $(C_1-C_4)$Alkyl, Halo-$(C_1-C_4)$alkyl, $(C_2-C_4)$Alkenyl, $(C_2-C_4)$Alkinyl, $(C_1-C_4)$Alkoxy, $[(C_1-C_2)$Alkoxy$]-(C_1-C_2)$alkyl, $(C_3-C_5)$Cycloalkyl oder einen Rest der Formel $NR^aR^b$, worin $R^a$ und $R^b$ unabhängig voneinander H oder $(C_1-C_4)$Alkyl sind,

$R^3$ $(C_1-C_4)$Alkyl, Halogen, Nitro oder $(C_1-C_4)$Alkoxy und

n 0 oder 1 bedeuten.

Weiterhin bevorzugt sind dabei erfindungsgemäße Verbindungen, worin $R^1$, H, $CH_3$ oder $C_2H_5$ und $R^2$ H, $(C_1-C_4)$Alkyl, $(C_1-C_2)$Haloalkyl, Vinyl, Cyclopropyl, Cyclobutyl, $(C_1-C_2)$Alkoxy oder $N(CH_3)_2$, insbesondere H, bedeuten.

Weiterhin bevorzugt sind erfindungsgemäße Verbindungen der Formel (I) oder deren Salze, worin

A ein Rest der Formel

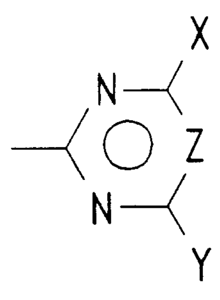

ist.

Vorzugsweise ist einer der Reste X und Y $(C_1-C_3)$Alkyl, $(C_1-C_3)$Alkoxy, $(C_1-C_3)$Haloalkyl, $(C_1-C_3)$-Haloalkoxy oder $[(C_1-C_2)$Alkoxy$]-(C_1-C_2)$alkyl und der andere Rest Y bzw. X $(C_1-C_3)$Alkyl, $(C_1-C_3)$Alkoxy, $(C_1-C_3)$Alkylthio, wobei jeder der letztgenannten 3 Reste unsubstituiert oder ein- oder mehrfach durch Halogen oder ein- oder zweifach durch $(C_1-C_3)$Alkoxy oder $(C_1-C_3)$Alkylthio substituiert ist, oder Halogen oder ein Rest der Formel $NR^5R^6$,worin $R^5$ und $R^6$ unabhängig voneinander H, $(C_1-C_3)$Alkyl oder $(C_3-C_4)$-

Alkenyl sind, oder $(C_3\text{-}C_6)$Cycloalkyl, $(C_2\text{-}C_4)$Alkenyl, $(C_2\text{-}C_4)$Alkinyl, $(C_3\text{-}C_4)$Alkenyloxy oder $(C_3\text{-}C_4)$-Alkinyloxy.

Noch mehr bevorzugt bedeutet einer der Reste X und Y $(C_1\text{-}C_2)$Alkyl, $(C_1\text{-}C_2)$Alkoxy oder $OCF_2H$ und der andere Rest Y bzw. X $(C_1\text{-}C_2)$Alkyl, $(C_1\text{-}C_2)$Alkoxy, Halogen, $OCF_2H$, $OCH_2CF_3$ oder $CF_3$.

Insbesondere sind X und Y unabhängig voneinander $(C_1\text{-}C_2)$Alkyl oder $(C_1\text{-}C_2)$Alkoxy.

Bevorzugt sind auch erfindungsgemäße Verbindungen der Formel (I) oder deren Salze, die eine Kombination von zwei oder mehreren der bevorzugt genannten Bedeutungen (Merkmale) aufweisen.

Weiterer Gegenstand der vorliegenden Erfindung sind Verfahren zur Herstellung der erfindungsgemäßen Verbindungen der Formel (I) oder deren Salze, dadurch gekennzeichnet, daß man

a) eine Verbindung der Formel (II),

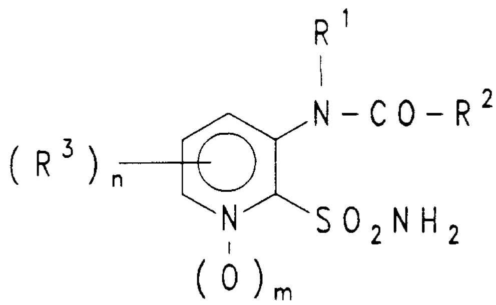

(II)

mit einem hetefocyclischen Carbamat der Formel (III),

$R^*$-O-CO-$NR^4$-A (III)

worin $R^*$ gegebenenfalls substituiertes Phenyl oder $(C_1\text{-}C_4)$Alkyl bedeutet, umsetzt oder

b) ein Pyridylsulfonylcarbamat der Formel (IV),

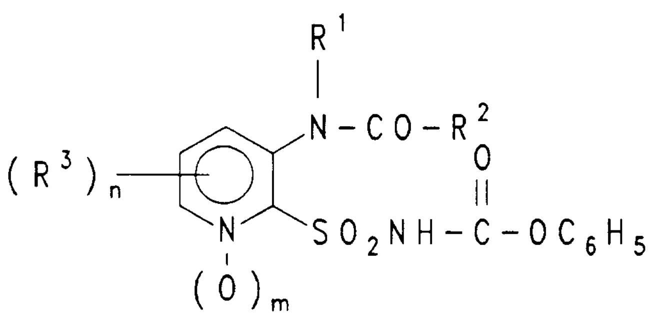

(IV)

mit einem Aminoheterocyclus der Formel (V)

H-$NR^4$-A (V)

umsetzt oder

c) ein Sulfonylisocyanat der Formel (VI)

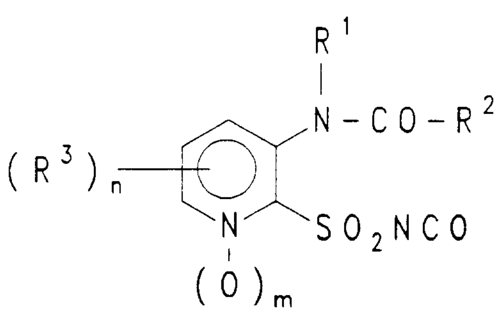

(VI)

mit einem Aminoheterocyclus der Formel (V)

H-$NR^4$-A (V)

umsetzt oder

d) in einer Eintopfreaktion zunächst einen Aminoheterocyclus der Formel H-$NR^4$-A (V) in Gegenwart einer Base, wie z.B. Triethylamin, mit Phosgen umsetzt und das gebildete Intermediat mit einem Pyridinsulfonamid der Formel (II) umsetzt,

wobei in den Formeln (II)-(VI) $R^1$, $R^2$, $R^3$, $R^4$, A, m und n wie in Formel I definiert sind.

Die Umsetzung der Verbindungen der Formeln (II) und (III) erfolgt vorzugsweise basenkatalysiert in einem inerten organischen Lösungsmittel, wie z. B. Dichlormethan, Acetonitril, Dioxan oder THF bei Temperaturen zwischen 0° C und dem Siedepunkt des Lösungsmittels. Als Base wird beispielsweise 1,8-Diazabicyclo-[5.4.0]undec-7-en (DBU) oder Trimethylaluminium oder Triethylaluminium verwendet.

Die Sulfonamide (II) sind neue Verbindungen. Sie und ihre Herstellung sind ebenfalls Gegenstand der Erfindung.

Man erhält die Verbindung der Formel (II) z. B. ausgehend von Verbindungen der Formel (VII)

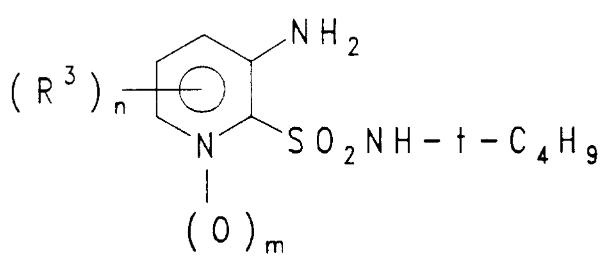

(VII)

durch Umsetzung mit Carbonsäurehalogeniden der Formel Hal-CO-$R^2$ (Hal = Halogen, vorzugsweise Chlor) oder symmetrischen oder gemischten Carbonsäureanhydriden der Formel $R^2$-CO-O-CO-$R^+$, worin $R^+$ analog $R^2$ definiert ist oder ein anderer aliphatischer oder aromatischer Rest ist, und anschließender Umsetzung des erhaltenen, mit der t-Butylgruppe geschützten 3-(N-Acylamino)-pyridin-2-sulfamids

a) mit einer starken Säure (z. B. Trifluoressigsäure) zum freien 3-(N-Acylamino)-pyridin-2-sulfamid der Formel (II), in der $R^1$ = H ist, oder

b) durch Reduktion mit z. B. Lithiumaluminiumhydrid (Reduktion der Acyl-Gruppe zur gegebenenfalls substituierten N-Alkylgruppe), darauffolgender, erneuter N-Acylierung mit einer Verbindung der Formel Hal-CO-$R^2$ oder $R^2$-CO-O-CO-$R^+$ und Abspaltung der t-Butyl-Schutzgruppe mit einer starken Säure (z. B. Trifluoressigsäure) zum freien 3-(N-Acylamino)-pyridin-2-sulfamid der Formel II, in der $R^1$ ungleich Wasserstoff ist.

Die einzelnen Reaktionsstufen können analog üblichen Verfahren durchgeführt werden. Die Sulfonamide der Formel (VII) sind nach literaturbekannten Verfahren aus 2-Chlor-3-nitropyridin bzw. 2-Chlor-3-amino-pyridin durch Umsetzung mit Schwefel-Nucleophilen, wie z. B. Benzylmercaptan, gegebenenfalls Reduktion der Nitrogruppe und anschließender oxidativer Chlorierung des Schwefelatoms mit Natriumhypochlorit oder Chlor (Bildung der Sulfochloride analog EP-A-272 855) sowie Umsetzung der erhaltenen Sulfochloride mit tert. Butylamin herstellbar.

Auf der Stufe der durch die tert. Butylgruppen geschützten Sulfonamide kann eine weitere Derivatisierung z. B. durch nucleophile Substitution mit S-Alkylverbindungen durchgeführt werden.

Alternativ können Verbindungen der Formel (II) aus 2-Chlor-3-amino-pyridin durch N-Acylierung und N-Alkylierung, Bildung der Sulfochloride wie vorstehend beschrieben und Umsetzung der Sulfochloride direkt mit Ammoniak nach oder analog üblichen Methoden hergestellt werden.

Die Carbamate der Formel (III) können nach Methoden hergestellt werden, die in den südafrikanischen Patentanmeldungen 82/5671 und 82/5045 bzw. EP-A 70804 (US-A-4 480 101) oder Research Disclosure RD 275056 beschrieben sind.

Die Umsetzung der Verbindungen (IV) mit den Aminoheterocyclen (V) führt man vorzugsweise in inerten, aprotischen Lösungsmitteln wie z. B. Dioxan, Acetonitril oder Tetrahydrofuran bei Temperaturen zwischen 0° C und der Siedetemperatur des Lösungsmittels durch. Die Pyridylsulfonylcarbamate der Formel (IV) erhält man analog EP-A-44 808 oder EP-A-237 292.

Die Pyridylsulfonylisocyanate der Formel (VI) lassen sich analog EP-A-184 385 herstellen und mit den Aminoheterocyclen der Formel (V) umsetzen.

Die Salze der Verbindungen der Formel (I) werden vorzugsweise in inerten Lösungsmitteln wie z. B. Wasser, Methanol oder Aceton bei Temperaturen von 0 bis 100° C hergestellt. Geeignete Basen zur Herstellung der erfindungsgemäßen Salze sind beispielsweise Alkalicarbonate, wie Kaliumcarbonat, Alkali- und Erdalkalihydroxide, Ammoniak oder Ethanolamin. Als Säuren zur Salzbildung eignen sich besonders HCl, HBr, $H_2SO_4$ oder $HNO_3$.

Mit den in den vorstehenden Verfahrensvarianten bezeichneten "inerten Lösungsmitteln" sind jeweils Lösungsmittel gemeint, die unter den jeweiligen Reaktionsbedingungen inert sind, jedoch nicht unter beliebigen Reaktionsbedingungen inert sein müssen.

Die erfindungsgemäßen Verbindungen der Formel (I) oder deren Salze weisen eine ausgezeichnete herbizide Wirksamkeit gegen ein breites Spektrum wirtschaftlicher wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielsweise einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt.

Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Verbindungen der Formel (I) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung, z.B. durch Auslösen von Desikkation und Wuchstauchung, eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen der Formel (I) können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) oder deren Salze enthalten.

Die Verbindungen der Formel (I) oder deren Salze können auf verschiedene Art formuliert werden, je nachdem welche biologischen und/oder chemischphysikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie
Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z. B. Sorbitanfettsäureester oder Polyoxethylensorbitanester wie z. B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z. B. oben bei den anderen Formulierungstypen bereits aufgeführt sind, hergestellt werden.

Emulsionen, z. B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z. B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdisbergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Die agrochemischen Zubereitungen enthalten in der Regel 0,1 bis 99 Gew.-%, insbesondere 0,1 bis 95 Gew.-%, Wirkstoff der Formel (I) oder deren Salze.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30, vorzugsweise meistens 5 bis 20 Gew.-% an Wirkstoff. versprühbare Lösungen etwa 0,05 bis 80, vorzugsweise 2 bis 50 Gew.-% Wirkstoff. Bei wasserdispergierbaren Granulaten hängt der Wirkstoffgehalt

zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26, 441-445 (1986), oder "The Pesticide Manual", 9th edition, The British Crop Protection Council, 1990/91, Bracknell, England, und dort zitierter Literatur beschrieben sind. Als literaturbekannte Herbizide, die mit den Verbindungen der Formel (I) kombiniert werden können, sind z. B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d. h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d. h. Ammoniumsulfamat; anilofos; asulam; atrazin; aziprotryn; barban; BAS 516 H, d. h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; carbetamide; CDAA, d. h. 2-Chlor-N,N-di-2-propenylacetamid; CDEC, d. h. Diethyldithiocarbaminsäure-2-chlorallylester; CGA 184927, d. h. 2-[4-[(5-Chlor-3-fluor-2-pyridinyl)-oxy]-phenoxy]-propansäure und 2-propynylester; chlomethoxyfen; chloramben; chlorazifop-butyl, pirifenop-butyl; chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clomazone; clomeprop; cloproxydim; clopyralid; cyanazine; cycloate; cycloxydim; cycluron; cyperquat; cyprazine; cyprazole; 2,4-DB; dalapon; desmediphan; desmetryn; di-allate; dicamba; dichlobenil; dichlorprop; diclofop-methyl; diethatyl; difenoxuron; difenzoquat; diflufenican; dimefuron; dimethachlor; dimethametryn; dimethazone, clomazon; dimethipin; dimetrasulfuron, cinosulfuron; dinitramine; dinoseb; dinoterb; diphenamid; dipropetryn; diquat; dithiopyr; diuron; DNOC; eglinazine-ethyl; EL 177, d. h. 5-Cyano-1-(1,1-dimethylethyl)-N-methyl-3H-pyrazole-4-carboxamid; endothal; EPTC; esprocarb; ethalfluralin; ethametsulfuron-methyl; ethidimuron; ethiozin; ethofumesate; F5231, d. h. N-[2-Chlor-4-fluor-5-[4-(3-fluorpropyl)-4,5-dihydro-5-oxo-1H-tetrazol-1-yl]-phenyl]-ethansulfonamid; F6285, d. h. 1-[5-(N-Methylsulfonyl)-amino-2,4-dichlorophenyl]-3-methyl-4-difluoromethyl-1,2,4-triazol-5-on; fenoprop; fenoxan, s. clomazon; fenoxaprop-ethyl; fenuron; flamprop-methyl; flazasulfuron; fluazifop und dessen Esterderivate; fluchloralin; flumetsulam; N-[2,6-Difluorphenyl]-5-methyl-(1,2,4)-triazolo[1,5a]pyrimidin-2-sulfonamid; flumeturon; flumipropyn; fluorodifen; fluoroglycofen-ethyl; fluridone; flurochloridone; fluroxypyr; flurtamone; fomesafen; fosamine; furyloxyfen; glufosinate; glyphosate; halosaten; haloxyfop und dessen Esterderivate; hexazinone; Hw 52, d. h. N-(2,3-Dichlorphenyl)-4-(ethoxymethoxy)-benzamid; imazamethabenz-methyl; imazapyr; imazaquin; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metobromuron; metolachlor; metoxuron; metribuzin; metsulfuron-methyl; MH; molinate; monalide; monocarbamide dihydrogensulfate; monolinuron; monuron; MT 128, d. h. 6-Chlor-N-(3-chlor-2-propenyl)-5-methyl-N-phenyl-3-pyridazinamin; MT 5950, d. h. N-[3-Chlor-4-(1-methylethyl]-phenyl]-2-methylpentanamid; naproanilide; napropamide; naptalam; NC 310, d. h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiazon; oxyfluorfen; paraquat; pebulate; pendimethalin; perfluidone; phenmedipham; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Esterderivate; propazine; propham; propyzamide; prosulfalin; prosulfocarb; prynachlor; pyrazolinate; pyrazon; pyrazosulfuron-ethyl; pyrazoxyfen; pyridate; quinclorac; quinmerac; quinofop und dessen Esterderivate, quizalofop und dessen Esterderivate; quizalofop-ethyl; quizalofop-p-tefuryl; renriduron; dymron; S 275, d. h. 2-[4-Chlor-2-fluor-5-(2-propynyloxy)-phenyl]-4,5,6,7-tetrahydro-2H-indazol; S 482, d. h. 2-[7-Fluor-3,4-dihydro-3-oxo-4-(2-propynyl)-2H-1,4-benzoxazin-6-yl]-4,5,6,7-tetrahydro-1H-isoindol-1,3(2H-dion; secbumeton; sethoxydim; siduron; simazine; simetryn; SN 106279, d. h. 2-[[7-[2-Chlor-4-(trifuormethyl)-phenoxy]-2-naphthalenyl]-oxy]-propansäure und -methylester; sulfometuron-methyl; sulfazuron; flazasulfuron; TCA; tebutam; tebuthiuron; terbacil; terbucarb; terbuchlor; terbumeton; terbuthylazine; terbutryn; TFH 450, d. h. N,N-Diethyl-3-[(2-ethyl-6-methylphenyl)-sulfonyl]-1H-1,2,4-triazol-1-carboxamid; thiazafluron; thifensulfuron-methyl; thiobencarb; tiocarbazil; tralkoxydim; tri-allate; triasulfuron; triazofenamide; tribenuron-methyl; triclopyr; tridiphane; trietazine; trifluralin; trimeturon; vernolate; WL 110547, d. h. 5-Phenoxy-1-[3-(trifluormethyl)-phenyl]-1H-tetrazol.

Zur Anwendung werden die in handelsüblicher Form vorliegenden Formulierungen gegebenenalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Die erfindungsgemäßen Verbindungen können beispielsweise direkt auf die Schadpflanzen oder Schadpflanzen und Nutzpflanzen zugleich im Nachauflaufverfahren oder auf die Fläche, auf der die Pflanzen wachsen, z.B. auf Ackerboden mit Pflanzensamen bzw. aufgelaufenen Pflanzen oder auf Anbauflächen, wie z.B. eine Reisanbaufläche, im Vor- oder Nachauflaufverfahren appliziert werden.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

A. Chemische Beispiele

Beispiele

a) 2-Benzylthio-3-nitropyridin

39,1 g (0,315 mol) Benzylmercaptan werden in 200 ml Acetonitril vorgelegt und mit 47,8 g (0,346 mol) $K_2CO_3$ 40 min bei 60° C gerührt. Anschließend tropft man 50,0 g (0,315 mol) 2-Chlor-3-nitropyridin in 150 ml Acetonitril gelöst hinzu und erhitzt 4 h zum Rückfluß. Das Acetonitril wird unter reduziertem Druck abdestilliert, der Rückstand in Dichlormethan aufgenommen, die organische Phase jeweils einmal mit gesättigter Natriumhydrogencarbonatlösung und mit 1 n Salzsäure gewaschen, die organische Phase über Magnesiumsulfat getrocknet, vom Trockenmittel abfiltriert und das Dichlormethan unter reduziertem Druck entfernt. Nach Umkristallisieren aus Methanol erhält man 60,9 g (79 % d. Th.) 2-Benzyl-3-nitropyridin vom Schmelzpunkt 72° C.

b) 3-Nitro-2-pyridip-tert.-butylsulfonamid

24,6 g (0,1 mol) 2-Benzylthio-3-nitropyridin werden in 360 ml $CH_2Cl_2$ gelöst. 280 ml Wasser hinzugefügt und bei 0° C 44,9 ml konz. HCl hinzugetropft. Anschließend läßt man 550 ml 5 %ige Natriumhypochloritlösung so langsam zulaufen, daß die Innentemperatur 5° C nicht überschreitet. Nach 30 min weiteren Rührens bei 0° C wird der Ansatz auf 500 ml Wasser gegeben, die organische Phase abgetrennt und die wäßrige Phase bei 0° C mit $CH_2Cl_2$ nachgewaschen. Die vereinigten organischen Phasen werden mit NaCl-Lösung gewaschen und über $MgSO_4$ getrocknet. Man tropft dann bei -70° C 32,1 g (0,44 mol) t-Butylamin hinzu. Man läßt den Ansatz auf Raumtemperatur aufwärmen, gießt in Wasser und stellt mit 1 n HCl auf pH 2-3. Nach Abtrennen der organischen Phase wird die wäßrige Phase mit $CH_2Cl_2$ nachgewaschen, die organischen Phasen vereinigt, getrocknet und das Lösungsmittel unter reduziertem Druck abgezogen. Nach Ausrühren des Rückstandes mit Diethylether und Abfiltrieren erhält man 14,7 g (57 % d. Th.) 3-Nitro-2-pyridin-tert.-butylsulfonamid vom Schmelzpunkt 134° C.

c) 3-Amino-2-pyridin-tert.-butylsulfonamid

14,7 g (0,057 mol) 3-Nitro-2-pyridin-tert.-butylsulfonamid werden in 49 ml Eisessig und 110 ml Wasser gelöst. Bei maximal 60° C werden langsam 15,8 g (0,28 mol) Eisenpulver hinzugefügt und 3 h bei 50° C gerührt. Nach Abkühlen wird das Eisenoxid abfiltriert und sowohl die wäßrige Phase als auch das Eisenoxid mehrfach mit $CH_2Cl_2$ gewaschen. Die vereinigten organischen Phasen werden getrocknet und das Lösungsmittel unter reduziertem Druck abgezogen. Man erhält 11,2 g (86 % d. Th.) 3-Amino-2-pyridin-tert.-butylsulfonamid vom Schmelzpunkt 163° C.

d) 3-Formylamino-2-pyridin-tert.-butylsulfonamid

0,48 g (0,01 mol) Ameisensäure und 1,3 g (0,013 mol) Essigsäureanhydrid werden 1 h bei 60° C gerührt. Dann fügt man 1,59 g (0,0069 mol) 3-Amino-2-pyridin-tert.-butylsulfonamid hinzu, rührt eine weitere Stunde bei 60° C, läßt auf Raumtemperatur abkühlen und erhält nach Ausrühren mit Ether 1,50 g (85 % d. Th.) 3-Formylamino-2-pyridin-tert.-butylsulfonamid vom Schmelzpunkt 168° C.

e) 3-Formylamino-2-pyridin-sulfonamid

1,50 g (0,0058 mol) 3-Formylamino-2-pyridin-tert.-butylsulfonamid werden 4 Tage bei Raumtemperatur in 20 ml wasserfreier Trifluoressigsäure gerührt. Die Trifluoressigsäure wird unter reduziertem Druck abgezogen und der Rückstand mit Ether ausgerührt. Man erhält 1,00 g (85 % d. Th.) 3-Formylamino-2-pyridinsulfonamid vom Schmelzpunkt 183° C.

f) N-[(4,6-Dimethoxypyrimidin-2-yl)-aminocarbonyl]-3-formyl-amino-2-pyridinsulfonamid (Beispiel 2 von Tabelle 1)

Zu einer Lösung aus 0,5 g (0,0019 mol) 3-Formylamino-2-pyridinsulfonamid und 0,68 g (0,0025 mol) N-(4,6-Dimethoxypyrimidin-2-yl)-phenylcarbamat in 40 ml Acetonitril gibt man 0,75 g (0,0049 mol) 1,8-Diazabicyclo[5.4.0]undec-7-en (DBU). Die Lösung wird 16 h bei Raumtemperatur gerührt, anschließend 20 ml Wasser hinzugefügt und mit 2 normaler Salzsäure auf pH 4 angesäuert. Man extrahiert mit Dichlormethan, trocknet die organische Phase über $MgSO_4$ und fügt n-Heptan hinzu. Nach Abfiltrieren erhält man 0,64 g (89 % d. Th.) N-[(4,6-Dimethoxy-pyrimidin-2-yl)-aminocarbonyl]-3-formylamino-2-pyridinsulfonamid vom Schmelzpunkt 172° C (Zers.).

g) N-[(2-Methoxy-4-methyl)-1,3,5-triazin-6-yl)]-amino-carbonyl-3-(N-methyl)acetylamino-2-pyridinsulfonamid

Zu 1,0 g (0,0044 mol) 3-(N-Methyl)acetylamino-2-pyridin-sulfonamid in 70 ml Dichlormethan werden 1,7 ml einer 20 %igen Lösung von Trimethylaluminium in Toluol zugetropft. Nach 15 min. Rühren bei Raumtemperatur fügt man 0,79 g (0,0044 mol) Methyl-2-methoxy-4-methyl-1,3,5-triazin-6-ylcarbamat in 20 ml Dichlormethan gelöst hinzu und erhitzt 20 h zum Rückfluß. Man läßt abkühlen, gießt in 60 ml eiskalte 1 normale Salzsäure und extrahiert die wäßrige Phase dreimal mit Dichlormethan. Nach Trocknen und Abziehen des Lösungsmittels erhält man 0,66 g (38 % d. Th.) N-[(2-Methoxy-4-methyl)-1,3,5-triazin-6-yl)-aminocarbonyl-3-(N-methylacetylamino)-2-pyridinsulfonamid vom Schmelzpunkt 165° C (Zers.).

Die anderen Verbindungen der nachfolgenden Tabellen 1-3 werden analog zu den Verfahren der Beispiele a bis g erhalten.

Tabelle 1

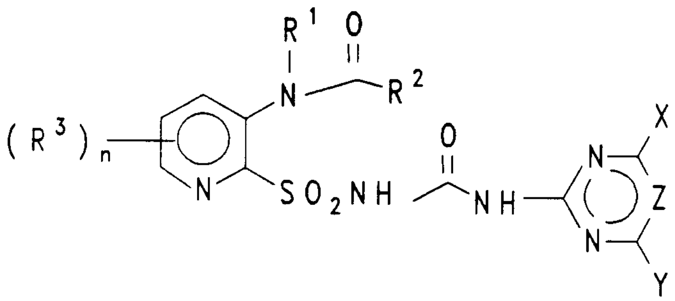

| Bsp. | $R^1$ | $R^2$ | $(R^3)_n$ | X | Y | Z | Smp. [°C] |
|---|---|---|---|---|---|---|---|
| 1 | H | $C_2H_5$ | - | $OCH_3$ | $OCH_3$ | CH | 138 |
| 2 | H | H | - | $OCH_3$ | $OCH_3$ | CH | 172 |
| 3 | H | H | - | $OC_2H_5$ | $OC_2H_5$ | CH | 166 |
| 4 | H | i-$C_3H_7$ | - | $OCH_3$ | $OCH_3$ | CH | 165 |
| 5 | H | $CF_3$ | - | $OCH_3$ | $OCH_3$ | CH | 175 |
| 6 | H | $OC_2H_5$ | - | $OCH_3$ | $OCH_3$ | CH | 171 |
| 7 | H | Cyclo-propyl | - | $OCH_3$ | $OCH_3$ | CH | 134 |
| 8 | H | $CH_3$ | - | $CH_3$ | $CH_3$ | CH | 182 |
| 9 | H | $N(CH_3)_2$ | - | $OCH_3$ | $OCH_3$ | CH | 182 |

| Bsp. | $R^1$ | $R^2$ | $(R^3)_n$ | X | Y | Z | Smp. [°C] |
|---|---|---|---|---|---|---|---|
| 10 | H | Phenyl | - | $OCH_3$ | $OCH_3$ | CH | 186 |
| 11 | H | $CH=CH_2$ | - | $OCH_3$ | $OCH_3$ | CH | 164 |
| 12 | H | Cyclobutyl | - | $OCH_3$ | $OCH_3$ | CH | 157 |
| 13 | H | $CH_2OCH_3$ | - | $OCH_3$ | $OCH_3$ | CH | 192 |
| 14 | H | $CH_2Cl$ | - | $OCH_3$ | $OCH_3$ | CH | 270 |
| 15 | H | $CH_2$-Br | - | $OCH_3$ | $OCH_3$ | CH | 207 |
| 16 | H | $CH_2$-$NO_2$ | - | $OCH_3$ | $OCH_3$ | CH | |
| 17 | H | $CH_2$-CN | - | $OCH_3$ | $OCH_3$ | CH | |
| 18 | H | N-$COC_3H_7$ with $C_3H_7$ on N | - | $OCH_3$ | $OCH_3$ | CH | 180 |
| 19 | $CH_3$ | $CH_3$ | - | $OCH_3$ | $OCH_3$ | CH | 180 |
| 20 | $CH_3$ | $C_2H_5$ | - | $OCH_3$ | $OCH_3$ | CH | 187 |
| 21 | $CH_3$ | $CF_3$ | - | $OCH_3$ | $OCH_3$ | CH | 142 |
| 22 | $CH_3$ | i-$C_3H_7$ | - | $OCH_3$ | $OCH_3$ | CH | 168 |
| 23 | $CH_3$ | $CH_3$ | - | $OCH_3$ | $CH_3$ | N | 165 |
| 24 | $CH_3$ | $CH_3$ | - | $CH_3$ | $CH_3$ | CH | 181 |
| 25 | $C_2H_5$ | H | - | $OCH_3$ | $OCH_3$ | CH | 185 |
| 26 | $CH_3$ | H | - | $OCH_3$ | $OCH_3$ | CH | 178 |
| 27 | $C_2H_5$ | H | - | $OCH_3$ | $CH_3$ | N | |
| 28 | $CH_3$ | H | - | $OCH_3$ | $CH_3$ | N | |
| 29 | n-$C_3H_7$ | H | - | $OCH_3$ | $OCH_3$ | CH | 184 |

| Bsp. | $R^1$ | $R^2$ | $(R^3)_n$ | X | Y | Z | Smp. [°C] |
|---|---|---|---|---|---|---|---|
| 30 | n-$C_3H_7$ | H | - | $OCH_3$ | $CH_3$ | N | |
| 31 | n-$C_3H_7$ | H | - | $CH_3$ | $CH_3$ | CH | |
| 32 | n-$C_4H_9$ | H | - | $OCH_3$ | $OCH_3$ | CH | 156 |
| 33 | n-$C_4H_9$ | H | - | $CH_3$ | $CH_3$ | CH | |
| 34 | n-$C_4H_9$ | H | - | $OCH_3$ | $CH_3$ | N | |
| 35 | i-$C_4H_9$ | H | - | $OCH_3$ | $OCH_3$ | CH | |
| 36 | i-$C_4H_9$ | H | - | $CH_3$ | $CH_3$ | CH | |
| 37 | i-$C_4H_9$ | H | - | $OCH_3$ | $CH_3$ | N | |
| 38 | Cyclobu-tylmethyl | H | - | $OCH_3$ | $OCH_3$ | CH | |
| 39 | Cyclopro-pylmethyl | H | - | $OCH_3$ | $OCH_3$ | CH | |
| 40 | $CH_3$ | $CH=CH_2$ | - | $OCH_3$ | $OCH_3$ | CH | |
| 41 | $CH_3$ | Phenyl | - | $OCH_3$ | $OCH_3$ | CH | |
| 42 | $CH_3$ | i-$C_4H_9$ | - | $OCH_3$ | $OCH_3$ | CH | 211 |
| 43 | $CH_3$ | $CH_2$-O-$CH_3$ | - | $OCH_3$ | $OCH_3$ | CH | |
| 44 | $CH_3$ | $CH_2$-Cl | - | $OCH_3$ | $OCH_3$ | CH | |
| 45 | $CH_3$ | $CH_2$-Br | - | $OCH_3$ | $OCH_3$ | CH | |
| 46 | $CH_3$ | $CH_2$-$NO_2$ | - | $OCH_3$ | $OCH_3$ | CH | |
| 47 | $CH_3$ | $CH_2$-CN | - | $OCH_3$ | $OCH_3$ | CH | |
| 48 | $CH_2$-$CF_3$ | $CH_3$ | - | $OCH_3$ | $OCH_3$ | CH | |

| Bsp. | $R^1$ | $R^2$ | $(R^3)_n$ | X | Y | Z | Smp. [°C] |
|---|---|---|---|---|---|---|---|
| 49 | $CH_2CF_3$ | $CF_3$ | - | $OCH_3$ | $OCH_3$ | CH | |
| 50 | $CH_2CF_3$ | H | - | $OCH_3$ | $OCH_3$ | CH | |
| 51 | Benzyl | $CH_3$ | - | $OCH_3$ | $OCH_3$ | CH | |
| 52 | Benzyl | $CF_3$ | - | $OCH_3$ | $OCH_3$ | CH | |
| 53 | Benzyl | H | - | $OCH_3$ | $OCH_3$ | CH | |
| 54 | $CH_3$ | $CH=CH_2$ | - | $CH_3$ | $CH_3$ | CH | |
| 55 | $CH_3$ | Phenyl | - | $CH_3$ | $CH_3$ | CH | |
| 56 | $CH_3$ | i-$C_4H_9$ | - | $CH_3$ | $CH_3$ | CH | |
| 57 | $CH_3$ | $CH_2$-$OCH_3$ | - | $CH_3$ | $CH_3$ | CH | |
| 58 | $CH_3$ | $CH_2$-Cl | - | $CH_3$ | $CH_3$ | CH | |
| 59 | $CH_3$ | $CH_2$-Br | - | $CH_3$ | $CH_3$ | CH | |
| 60 | $CH_3$ | $CH_2NO_2$ | - | $CH_3$ | $CH_3$ | CH | |
| 61 | $CH_3$ | $CH_2$-CN | - | $CH_3$ | $CH_3$ | CH | |
| 62 | $CH_2$-$CF_3$ | $CH_3$ | - | $CH_3$ | $CH_3$ | CH | |
| 63 | $CH_2$-$CF_3$ | $CF_3$ | - | $CH_3$ | $CH_3$ | CH | |
| 64 | $CH_2$-$CF_3$ | H | - | $CH_3$ | $CH_3$ | CH | |
| 65 | Benzyl | $CH_3$ | - | $CH_3$ | $CH_3$ | CH | |
| 66 | Benzyl | $CF_3$ | - | $CH_3$ | $CH_3$ | CH | |
| 67 | Benzyl | H | - | $CH_3$ | $CH_3$ | CH | |
| 68 | $CH_3$ | $CH=CH_2$ | - | $OCH_3$ | $CH_3$ | N | |
| 69 | $CH_3$ | Phenyl | - | $OCH_3$ | $CH_3$ | N | |

| Bsp. | $R^1$ | $R^2$ | $(R^3)_n$ | X | Y | Z | Smp. [°C] |
|---|---|---|---|---|---|---|---|
| 70 | $CH_3$ | i-$C_4H_9$ | - | $OCH_3$ | $CH_3$ | N | |
| 71 | $CH_3$ | $CH_2$-$OCH_3$ | - | $OCH_3$ | $CH_3$ | N | |
| 72 | $CH_3$ | $CH_2$-Cl | - | $OCH_3$ | $CH_3$ | N | |
| 73 | $CH_3$ | $CH_2$-Br | - | $OCH_3$ | $CH_3$ | N | |
| 74 | $CH_3$ | $CH_2$-$NO_2$ | - | $OCH_3$ | $CH_3$ | N | |
| 75 | $CH_3$ | $CH_2$-CN | - | $OCH_3$ | $CH_3$ | N | |
| 76 | $CH_2$-$CF_3$ | $CH_3$ | - | $OCH_3$ | $CH_3$ | N | |
| 77 | $CH_2$-$CF_3$ | $CF_3$ | - | $OCH_3$ | $CH_3$ | N | |
| 78 | $CH_2$-$CF_3$ | H | - | $OCH_3$ | $CH_3$ | N | |
| 79 | Benzyl | $CH_3$ | - | $OCH_3$ | $CH_3$ | N | |
| 80 | Benzyl | $CF_3$ | - | $OCH_3$ | $CH_3$ | N | |
| 81 | Benzyl | H | - | $OCH_3$ | $CH_3$ | N | |
| 82 | $C_2H_5$ | $CH_3$ | - | $OCH_3$ | $OCH_3$ | CH | 185 |
| 83 | $C_2H_5$ | $CH_3$ | - | $CH_3$ | $CH_3$ | CH | |
| 84 | $C_2H_5$ | $CH_3$ | - | $OCH_3$ | $CH_3$ | N | |
| 85 | $C_2H_5$ | H | - | $CH_3$ | $CH_3$ | CH | |
| 86 | $C_2H_5$ | $CF_3$ | - | $OCH_3$ | $OCH_3$ | CH | 190 |
| 87 | $C_2H_5$ | $CF_3$ | - | $CH_3$ | $CH_3$ | CH | |
| 88 | $C_2H_5$ | $CF_3$ | - | $OCH_3$ | $CH_3$ | N | |
| 89 | $C_2H_5$ | $CH=CH_2$ | - | $OCH_3$ | $OCH_3$ | CH | |
| 90 | $C_2H_5$ | $CH=CH_2$ | - | $CH_3$ | $CH_3$ | CH | |

| Bsp. | $R^1$ | $R^2$ | $(R^3)_n$ | X | Y | Z | Smp. [°C] |
|---|---|---|---|---|---|---|---|
| 91 | $C_2H_5$ | $CH=CH_2$ | - | $OCH_3$ | $CH_3$ | N | |
| 92 | $C_2H_5$ | $CH_2$-$OCH_3$ | - | $OCH_3$ | $OCH_3$ | CH | |
| 93 | $C_2H_5$ | $CH_2$-$OCH_3$ | - | $CH_3$ | $CH_3$ | CH | |
| 94 | $C_2H_5$ | $CH_2$-O-$CH_3$ | - | $OCH_3$ | $CH_3$ | N | |
| 95 | $C_2H_5$ | $CH_2$-Cl | - | $OCH_3$ | $OCH_3$ | CH | |
| 96 | $C_2H_5$ | $CH_2$-Cl | - | $CH_3$ | $CH_3$ | CH | |
| 97 | $C_2H_5$ | $CH_2$-Cl | - | $OCH_3$ | $CH_3$ | N | |
| 98 | $C_2H_5$ | $CH_2$-Br | - | $OCH_3$ | $OCH_3$ | CH | |
| 99 | $C_2H_5$ | $CH_2$-Br | - | $CH_3$ | $CH_3$ | CH | |
| 100 | $C_2H_5$ | $CH_2$-Br | - | $OCH_3$ | $CH_3$ | N | |
| 101 | $C_2H_5$ | $CH_2$-$NO_2$ | - | $OCH_3$ | $OCH_3$ | CH | |
| 102 | $C_2H_5$ | $CH_2$-$NO_2$ | - | $CH_3$ | $CH_3$ | CH | |
| 103 | $C_2H_5$ | $CH_2$-$NO_2$ | - | $OCH_3$ | $CH_3$ | N | |
| 104 | $C_2H_5$ | $CH_2$-CN | - | $OCH_3$ | $OCH_3$ | CH | |
| 105 | $C_2H_5$ | $CH_2$-CN | - | $CH_3$ | $CH_3$ | CH | |
| 106 | $C_2H_5$ | $CH_2$-CN | - | $OCH_3$ | $CH_3$ | N | |
| 107 | $C_2H_5$ | i-$C_4H_9$ | - | $OCH_3$ | $OCH_3$ | CH | |
| 108 | $C_2H_5$ | i-$C_4H_9$ | - | $CH_3$ | $CH_3$ | CH | |
| 109 | $C_2H_5$ | i-$C_4H_9$ | - | $OCH_3$ | $CH_3$ | N | |
| 110 | H | $C_2H_5$ | 6-$CH_3$ | $OCH_3$ | $OCH_3$ | CH | |
| 111 | H | H | 6-$CH_3$ | $OCH_3$ | $OCH_3$ | CH | |

| Bsp. | $R^1$ | $R^2$ | $(R^3)_n$ | X | Y | Z | Smp. [°C] |
|---|---|---|---|---|---|---|---|
| 112 | H | H | 6-$CH_3$ | $OC_2H_5$ | $OC_2H_5$ | CH | |
| 113 | H | i-$C_3H_7$ | 6-$CH_3$ | $OCH_3$ | $OCH_3$ | CH | |
| 114 | H | $CF_3$ | 6-$CH_3$ | $OCH_3$ | $OCH_3$ | CH | |
| 115 | H | $OC_2H_5$ | 6-$CH_3$ | $OCH_3$ | $OCH_3$ | CH | |
| 116 | H | Cyclopropyl | 6-$CH_3$ | $OCH_3$ | $OCH_3$ | CH | |
| 117 | H | $CH_3$ | 6-$CH_3$ | $CH_3$ | $CH_3$ | CH | |
| 118 | H | $N(CH_3)_2$ | 6-$CH_3$ | $OCH_3$ | $OCH_3$ | CH | |
| 119 | H | Phenyl | 6-$CH_3$ | $OCH_3$ | $OCH_3$ | CH | |
| 120 | H | $CH=CH_2$ | 6-$CH_3$ | $OCH_3$ | $OCH_3$ | CH | |
| 121 | H | Cyclobutyl | 6-$CH_3$ | $OCH_3$ | $OCH_3$ | CH | |
| 122 | H | $CH_2$-O-$CH_3$ | 6-$CH_3$ | $OCH_3$ | $OCH_3$ | CH | |
| 123 | H | $CH_2$-Cl | 6-$CH_3$ | $OCH_3$ | $OCH_3$ | CH | |
| 124 | H | $CH_2$-Br | 6-$CH_3$ | $OCH_3$ | $OCH_3$ | CH | |
| 125 | H | $CH_2$-CN | 6-$CH_3$ | $OCH_3$ | $OCH_3$ | CH | |
| 126 | $CH_3$ | $CH_3$ | 6-$CH_3$ | $OCH_3$ | $OCH_3$ | CH | |
| 127 | $CH_3$ | $C_2H_5$ | 6-$CH_3$ | $OCH_3$ | $OCH_3$ | CH | |
| 128 | $CH_3$ | $CF_3$ | 6-$CH_3$ | $OCH_3$ | $OCH_3$ | CH | |
| 129 | $CH_3$ | i-$C_3H_7$ | 6-$CH_3$ | $OCH_3$ | $OCH_3$ | CH | |
| 130 | $CH_3$ | $CH_3$ | 6-$CH_3$ | $OCH_3$ | $CH_3$ | N | |
| 131 | $CH_3$ | $CH_3$ | 6-$CH_3$ | $CH_3$ | $CH_3$ | CH | |
| 132 | $C_2H_5$ | H | 6-$CH_3$ | $OCH_3$ | $OCH_3$ | CH | |

| Bsp. | $R^1$ | $R^2$ | $(R^3)_n$ | X | Y | Z | Smp. [°C] |
|---|---|---|---|---|---|---|---|
| 133 | $CH_3$ | H | 6-$CH_3$ | $OCH_3$ | $OCH_3$ | CH | |
| 134 | $C_2H_5$ | H | 6-$CH_3$ | $OCH_3$ | $CH_3$ | N | |
| 135 | $CH_3$ | H | 6-$CH_3$ | $OCH_3$ | $CH_3$ | N | |
| 136 | n-$C_3H_7$ | H | 6-$CH_3$ | $OCH_3$ | $OCH_3$ | CH | |
| 137 | n-$C_3H_7$ | H | 6-$CH_3$ | $OCH_3$ | $CH_3$ | N | |
| 138 | n-$C_4H_9$ | H | 6-$CH_3$ | $OCH_3$ | $OCH_3$ | CH | |
| 139 | n-$C_4H_9$ | H | 6-$CH_3$ | $CH_3$ | $CH_3$ | CH | |
| 140 | n-$C_4H_9$ | H | 6-$CH_3$ | $OCH_3$ | $CH_3$ | N | |
| 141 | i-$C_4H_9$ | H | 6-$CH_3$ | $OCH_3$ | $OCH_3$ | CH | |
| 142 | i-$C_4H_9$ | H | 6-$CH_3$ | $CH_3$ | $CH_3$ | CH | |
| 143 | i-$C_4H_9$ | H | 6-$CH_3$ | $OCH_3$ | $CH_3$ | N | |
| 144 | Cyclobutylmethyl | H | 6-$CH_3$ | $OCH_3$ | $OCH_3$ | CH | |
| 145 | Cyclopropylmethyl | H | 6-$CH_3$ | $OCH_3$ | $OCH_3$ | CH | |
| 146 | $CH_3$ | $CH=CH_2$ | 6-$CH_3$ | $OCH_3$ | $OCH_3$ | CH | |
| 147 | $CH_3$ | $CH_2$-O-$CH_3$ | 6-$CH_3$ | $OCH_3$ | $OCH_3$ | CH | |
| 148 | $CH_2$-$CF_3$ | $CH_3$ | 6-$CH_3$ | $OCH_3$ | $OCH_3$ | CH | |
| 149 | $CH_2$-$CF_3$ | $CF_3$ | 6-$CH_3$ | $OCH_3$ | $OCH_3$ | CH | |
| 150 | $CH_2$-$CF_3$ | H | 6-$CH_3$ | $OCH_3$ | $OCH_3$ | CH | |
| 151 | H | H | 6-Cl | $OCH_3$ | $OCH_3$ | CH | |

| Bsp. | $R^1$ | $R^2$ | $(R^3)_n$ | X | Y | Z | Smp. [°C] |
|---|---|---|---|---|---|---|---|
| 152 | $CH_3$ | H | 6-Cl | $OCH_3$ | $OCH_3$ | CH | |
| 153 | $C_2H_5$ | H | 6-Cl | $OCH_3$ | $OCH_3$ | CH | |
| 154 | n-$C_3H_7$ | H | 6-Cl | $OCH_3$ | $OCH_3$ | CH | |
| 155 | n-$C_4H_9$ | H | 6-Cl | $OCH_3$ | $OCH_3$ | CH | |
| 156 | H | $CH_3$ | 6-Cl | $OCH_3$ | $OCH_3$ | CH | |
| 157 | H | $C_2H_5$ | 6-Cl | $OCH_3$ | $OCH_3$ | CH | |
| 158 | H | i-$C_3H_7$ | 6-Cl | $OCH_3$ | $OCH_3$ | CH | |
| 159 | H | Phenyl | 6-Cl | $OCH_3$ | $OCH_3$ | CH | |
| 160 | H | $CH=CH_2$ | 6-Cl | $OCH_3$ | $OCH_3$ | CH | |
| 161 | H | $CH_2$-$OCH_3$ | 6-Cl | $OCH_3$ | $OCH_3$ | CH | |
| 162 | H | $CH_2$-Cl | 6-Cl | $OCH_3$ | $OCH_3$ | CH | |
| 163 | H | $CF_3$ | 6-Cl | $OCH_3$ | $OCH_3$ | CH | |
| 164 | H | $CH_2$-CN | 6-Cl | $OCH_3$ | $OCH_3$ | CH | |
| 165 | H | $CH_2$-$NO_2$ | 6-Cl | $OCH_3$ | $OCH_3$ | CH | |
| 166 | $CH_3$ | $CH_3$ | 6-Cl | $OCH_3$ | $OCH_3$ | CH | |
| 167 | $CH_3$ | $CF_3$ | 6-Cl | $OCH_3$ | $OCH_3$ | CH | |
| 168 | $CH_3$ | H | 6-$CF_3$ | $OCH_3$ | $OCH_3$ | CH | |
| 169 | $CH_3$ | $CH_2$-$OCH_3$ | 6-Cl | $OCH_3$ | $OCH_3$ | CH | |
| 170 | $C_2H_5$ | H | 6-$CF_3$ | $OCH_3$ | $OCH_3$ | CH | |
| 171 | $C_2H_5$ | $CH_3$ | 6-Cl | $OCH_3$ | $OCH_3$ | CH | |
| 172 | $C_2H_5$ | $CF_3$ | 6-Cl | $OCH_3$ | $OCH_3$ | CH | |

| Bsp. | $R^1$ | $R^2$ | $(R^3)_n$ | X | Y | Z | Smp. [°C] |
|---|---|---|---|---|---|---|---|
| 173 | $C_2H_5$ | $CH_2$-$OCH_3$ | 6-Cl | $OCH_3$ | $OCH_3$ | CH | |
| 174 | H | H | 6-$OCH_3$ | $OCH_3$ | $OCH_3$ | CH | |
| 175 | $CH_3$ | H | 6-$OCH_3$ | $OCH_3$ | $OCH_3$ | CH | |
| 176 | $C_2H_5$ | H | 6-$OCH_3$ | $OCH_3$ | $OCH_3$ | CH | |
| 177 | H | $CH_3$ | 6-$OCH_3$ | $OCH_3$ | $OCH_3$ | CH | |
| 178 | H | $C_2H_5$ | 6-$OCH_3$ | $OCH_3$ | $OCH_3$ | CH | |
| 179 | H | $CF_3$ | 6-$OCH_3$ | $OCH_3$ | $OCH_3$ | CH | |
| 180 | $CH_2CF_3$ | H | 6-$OCH_3$ | $OCH_3$ | $OCH_3$ | CH | |
| 181 | $CH_2CF_3$ | $CH_3$ | 6-$OCH_3$ | $OCH_3$ | $OCH_3$ | CH | |
| 182 | H | $CH=CH_2$ | 6-$OCH_3$ | $OCH_3$ | $OCH_3$ | CH | |
| 183 | $CH_3$ | $CH_3$ | 6-$CF_3$ | $OCH_3$ | $OCH_3$ | CH | |
| 184 | $CH_3$ | $CH_3$ | - | $OCHF_2$ | $OCHF_2$ | CH | |
| 185 | $CH_3$ | $CH_3$ | 6-Cl | $OCHF_2$ | $OCHF_2$ | CH | |
| 186 | $C_2H_5$ | H | - | $OC_2H_5$ | $OC_2H_5$ | CH | 147 |
| 187 | H | CO-$OCH_3$ | - | $OCH_3$ | $OCH_3$ | CH | 144 |
| 188 | H | $CHCl_2$ | - | $OCH_3$ | $OCH_3$ | CH | 116 |
| 189 | H | $CCl=CCl_2$ | - | $OCH_3$ | $OCH_3$ | CH | 174 |
| 190 | H | $CCl=CCl_2$ | - | $CH_3$ | $CH_3$ | CH | 241 |
| 191 | $CH_3$ | $CF_3$ | - | $CH_3$ | $CH_3$ | CH | 186 |
| 192 | $CH_3$ | $OC_2H_5$ | - | $OCH_3$ | $OCH_3$ | CH | 175 |
| 193 | H | $OC_2H_5$ | 6-F | $OCH_3$ | $OCH_3$ | CH | 176 |

| Bsp. | $R^1$ | $R^2$ | $(R^3)_n$ | X | Y | Z | Smp. [°C] |
|---|---|---|---|---|---|---|---|
| 194 | H | i-$C_3H_7$ | 6-F | $OCH_3$ | $OCH_3$ | CH | 187 |
| 195 | $CH_3$ | Cyclopropyl | - | $OCH_3$ | $OCH_3$ | CH | 187 |
| 196 | H | $CCl_3$ | - | $OCH_3$ | $OCH_3$ | CH | 160 |
| 197 | $CH_3$ | Cyclobutyl | - | $OCH_3$ | $OCH_3$ | CH | 207 |
| 198 | $CH_2$-$OCH_3$ | H | - | $OCH_3$ | $OCH_3$ | CH | 128 |
| 199 | H | $OCH_3$ | - | $OCH_3$ | $OCH_3$ | CH | 200 |
| 200 | $C_2H_5$ | $CCl_3$ | - | $OCH_3$ | $OCH_3$ | CH | 196 |
| 201 | $CH_3$ | $OCH_3$ | - | $OCH_3$ | $OCH_3$ | CH | 178 |
| 202 | $CH_3$ | $CH_2Cl$-$C(CH_3)_2Cl$ | - | $OCH_3$ | $OCH_3$ | CH | 222 |
| 203 | H | $CH_2Cl$-$C(CH_3)_2Cl$ | - | $OCH_3$ | $OCH_3$ | CH | 175 |
| 204 | H | $NC_2H_5$ | - | $OCH_3$ | $OCH_3$ | CH | 132 |
| 205 | $C_2H_5$ | $OCH_3$ | - | $OCH_3$ | $OCH_3$ | CH | 199 |
| 206 | $C_2H_5$ | $CHCl_2$ | - | $OCH_3$ | $OCH_3$ | CH | 181 |
| 207 | $CH_3$ | $CHCl_2$ | - | $OCH_3$ | $OCH_3$ | CH | 176 |
| 208 | H | $CH_2$-$(CF_2)_3CF_3$ | - | $OCH_3$ | $OCH_3$ | CH | 239 |
| 209 | $CH_3$ | $CCl=CCl_2$ | - | $OCH_3$ | $OCH_3$ | CH | 143 |

Tabelle 2

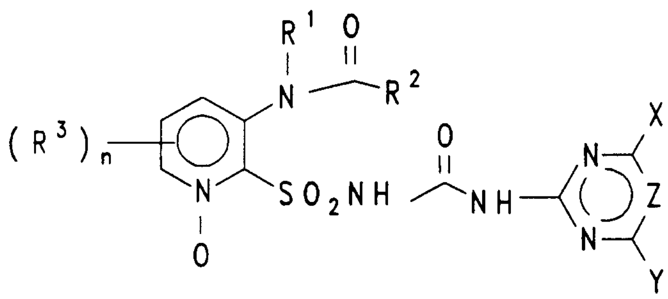

| Beispiel | $R^1$ | $R^2$ | $(R^3)_n$ | X | Y | Z | Smp. |
|---|---|---|---|---|---|---|---|
| 210 | H | H | - | $OCH_3$ | $OCH_3$ | CH | |
| 211 | H | $CH_3$ | - | $OCH_3$ | $OCH_3$ | CH | |
| 212 | H | $CH_2CH_3$ | - | $OCH_3$ | $OCH_3$ | CH | |
| 213 | H | $CH_2CH(CH_3)_2$ | - | $OCH_3$ | $OCH_3$ | CH | |
| 214 | H | $CF_3$ | - | $OCH_3$ | $OCH_3$ | CH | |
| 215 | $CH_3$ | H | - | $OCH_3$ | $OCH_3$ | CH | |
| 216 | $CH_3$ | $CH_3$ | - | $OCH_3$ | $OCH_3$ | CH | |
| 217 | $CH_3$ | $CH_2CH_3$ | - | $OCH_3$ | $OCH_3$ | CH | |
| 218 | $CH_3$ | $CH_2CH(CH_3)_2$ | - | $OCH_3$ | $OCH_3$ | CH | |
| 219 | $C_2H_5$ | H | - | $OCH_3$ | $OCH_3$ | CH | |
| 220 | $C_2H_5$ | $CH_3$ | - | $OCH_3$ | $OCH_3$ | CH | |
| 221 | $C_2H_5$ | $C_2H_5$ | - | $OCH_3$ | $OCH_3$ | CH | |
| 222 | $C_2H_5$ | $CF_3$ | - | $OCH_3$ | $OCH_3$ | CH | |

Tabelle 3

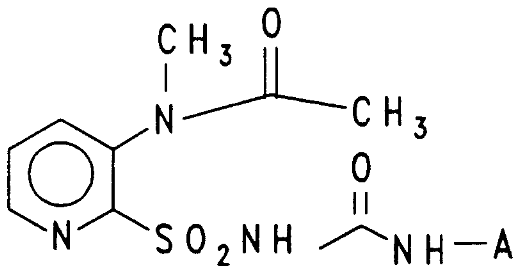

| Beispiel | A | Smp. |
|---|---|---|
| 223 | 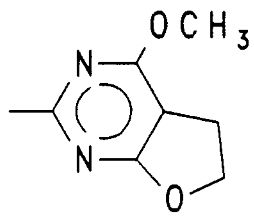 | |
| 224 | 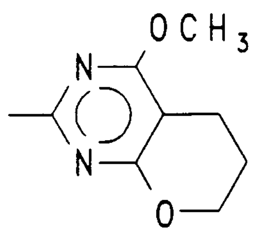 | |
| 225 | 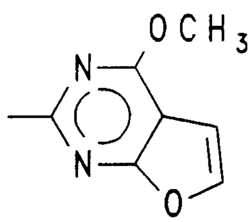 | |
| 226 | 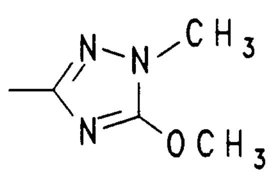 | |

| Beispiel | A | Smp. |
|---|---|---|
| 227 | 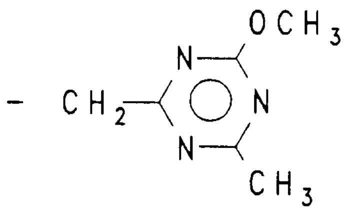 | |
| 228 | 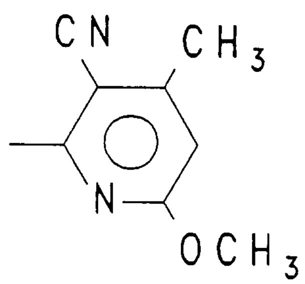 | |
| 229 | 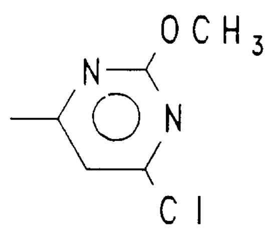 | |

Tabelle 4

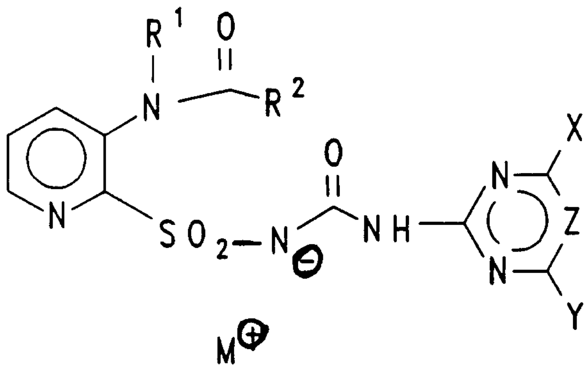

| Beispiel | $R^1$ | $R^2$ | M⊕ | X | Y | Z | Smp. |
|---|---|---|---|---|---|---|---|
| 230 | $CH_3$ | i-$C_3H_7$ | Na+ | $OCH_3$ | $OCH_3$ | CH | 191°C |
| 231 | $C_2H_5$ | H | Na+ | $OCH_3$ | $OCH_3$ | CH | |
| 232 | $CH_3$ | $CH_3$ | $NH_4$+ | $OCH_3$ | $OCH_3$ | CH | |
| 233 | $CH_3$ | $CH_3$ | Na+ | $OCH_3$ | $OCH_3$ | CH | |
| 234 | $C_2H_5$ | H | $NH_4$+ | $OCH_3$ | $OCH_3$ | CH | |
| 235 | $C_2H_5$ | $CH_3$ | Li+ | $OCH_3$ | $OCH_3$ | CH | |
| 236 | $C_2H_5$ | H | K+ | $OCH_3$ | $OCH_3$ | CH | |
| 237 | $CH_3$ | $CF_3$ | Na+ | $OCH_3$ | $OCH_3$ | CH | |

B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.

b) Ein in Wasser leicht dispergierbares, benetzbares Pulver wird enthalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel micht und in einer Stiftmühle mahlt.

c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 ° C) mischt und in einer Reibkugelmühle auf eine Feinheit von unter 5 Mikron vermahlt.

d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.

e) Ein in Wasser dispergierbares Granulat wird erhalten indem man

| | | |
|---|---|---|
| 75 Gewichtsteile | einer Verbindung der Formel (I), |
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.

f) Ein in Wasser dispergierbares Granulat wird auch erhalten, indem man

| | | |
|---|---|---|
| 25 Gewichtsteile | einer Verbindung der Formel (I), |
| 5 | " | 2,2'-dinaphthylmethan-6,6'-disulfonsaures Natrium, |
| 2 | " | oleoylmethyltaurinsaures Natrium, |
| 1 Gewichtsteil | | Polyvinylalkohol, |

| | | |
|---|---|---|
| 17 Gewichtsteile | | Calciumcarbonat und |
| 50 | " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

C. Biologische Beispiele

1. Unkrautwirkung im Vorauflauf

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspension bzw. Emulsion mit einer Wasseraufwandmenge von umgerechnet 600 bis 800 l/ha in unterschiedlichen Dosierungen auf die Oberfläche der Abdeckerde appliziert.

Nach der Behandlung wurden die Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Verbindungen der Beispiele 1, 2, 3, 4, 5, 6, 7, 8, 9, 10, 11, 12, 13, 14, 15, 18, 19, 20, 21, 22, 24, 25, 26, 29, 32, 42, 82, 86, 186-209 und 230 sehr gute herbizide Wirkung gegen Schadplanzen wie Sinapis alba, Chrysanthemum segetum, Avena sativa, Stellaria media, Echinochloa crus-galli, Lolium multiflorum, Setaria spp., Abutilon theophrasti, Amaranthus retroflexus und Panicum miliaceum im Vorauflaufverfahren bei einer Aufwandmenge von 0,3 kg und weniger Aktivsubstanz pro Hektar.

2. Unkrautwirkung im Nachauflauf

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumsbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha

auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Verbindungen der Beispiele 1, 2, 3, 4, 5, 6, 7, 8, 9, 10, 11, 12, 13, 14, 15, 18, 19, 20, 21, 22, 24, 25, 26, 29, 32, 42, 82, 86, 186-209 und 230 sehr gute herbizide Wirkung gegen Schadplanzen wie Sinapis alba, Stellaria media, Echinochloa crus-galli, Lolium multiflorum, Chrysanthemum segetum, Setaria spp., Abutilon theophrasti, Amaranthus retroflexus und Panicum miliaceum, Avena sativa im Nachauflaufverfahren bei einer Aufwandmenge von 0,3 kg und weniger Aktivsubstanz pro Hektar.

## Patentansprüche

**1.** Verbindungen der Formel (I) oder deren Salze,

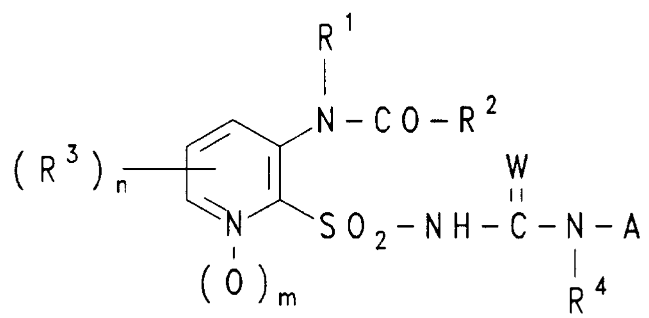

(I)

worin

| | |
|---|---|
| $R^1$ | H, $(C_1\text{-}C_6)$Alkyl, das unsubstituiert oder durch einen oder mehrere Reste aus der Gruppe Halogen, Nitro, $(C_1\text{-}C_4)$Alkoxy, $(C_3\text{-}C_6)$Cycloalkyl, Aryl und substituiertes Aryl substituiert ist, oder Aryl, wobei der Arylrest unsubstituiert oder durch einen oder mehrere Reste aus der Gruppe Halogen, Nitro, $(C_1\text{-}C_4)$Alkyl, $(C_1\text{-}C_4)$Haloalkyl und $(C_1\text{-}C_4)$Alkoxy substituiert ist, |
| $R^2$ | H, $(C_1\text{-}C_6)$Alkyl, $(C_2\text{-}C_6)$Alkenyl oder $(C_2\text{-}C_6)$Alkinyl, wobei jeder der drei letztgenannten Reste unsubstituiert oder durch einen oder mehrere Reste aus der Gruppe Halogen, $(C_1\text{-}C_4)$Alkoxy, $(C_1\text{-}C_4)$Alkylthio, $(C_1\text{-}C_4)$Alkylsulfinyl, $(C_1\text{-}C_4)$-Alkylsulfonyl, Nitro, Cyano und Thiocyanato substituiert ist, oder $(C_1\text{-}C_6)$Alkoxy oder $(C_1\text{-}C_6)$Alkylthio, wobei die letztgenannten 2 Reste unsubstituiert oder durch einen oder mehrere Reste aus der Gruppe Halogen, $(C_1\text{-}C_4)$Alkoxy, $(C_1\text{-}C_4)$Alkylthio, Aryl und substituiertes Aryl substituiert sind, $(C_3\text{-}C_7)$Cycloalkyl oder $(C_3\text{-}C_7)$-Cycloalkoxy, wobei die letztgenannten 2 Reste unsubstituiert oder durch ein oder mehrere Reste aus der Gruppe $(C_1\text{-}C_4)$Alkyl, $(C_1\text{-}C_4)$-Alkoxy, $(C_1\text{-}C_4)$Alkylthio, $(C_1\text{-}C_4)$Haloalkyl und Halogen substituiert sind, oder Aryl, substituiertes Aryl oder einen Rest der Formel $NR^aR^b$, |
| $R^3$ | $(C_1\text{-}C_4)$Alkyl, $(C_1\text{-}C_3)$Haloalkyl, Halogen, $NO_2$, CN, $(C_1\text{-}C_3)$Alkoxy, $(C_1\text{-}C_3)$Haloalkoxy, $(C_1\text{-}C_3)$Alkylthio, $(C_1\text{-}C_3)$Alkoxy-$(C_1\text{-}C_3)$alkyl, $[(C_1\text{-}C_3)$-Alkoxy]-carbonyl, $(C_1\text{-}C_3)$Alkylamino, Di$[(C_1\text{-}C_3)$alkyl]-amino, $(C_1\text{-}C_6)$-Alkylsulfinyl, $(C_1\text{-}C_3)$Alkylsulfonyl, $SO_2NR^cR^d$ oder $C(O)NR^eR^f$, |
| $R^a$, $R^b$, $R^c$, $R^d$, $R^e$ und $R^f$ | unabhängig voneinander H, $(C_1\text{-}C_4)$Alkyl, $(C_3\text{-}C_6)$Alkenyl,$(C_3\text{-}C_6)$Alkinyl, $[(C_1\text{-}C_4]$Alkyl]-carbonyl, Arylcarbonyl, das im Arylrest unsubstituiert oder substituiert ist, oder paarweise $R^a$ und $R^b$, $R^c$ und $R^d$ bzw. $R^e$ und $R^f$ gemeinsam mit dem sie verbindenden N-Atom einen heterocyclischen gesättigten oder ungesättigten Ring mit 3 bis 7 Ringatomen und 0, 1 oder 2 weiteren Heteroatomen aus der Gruppe N, O und S, der unsubstituiert oder substituiert ist, |
| $R^4$ | H oder $(C_1\text{-}C_4)$Alkyl, |
| m | 0 oder 1, |

n 0, 1 oder 2,

A einen Rest der Formel

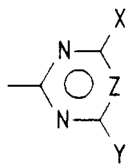 , 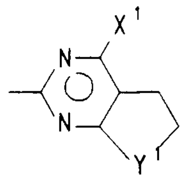 , 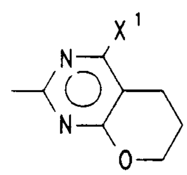 ,

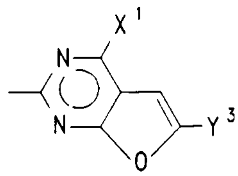 , 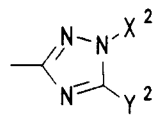 , 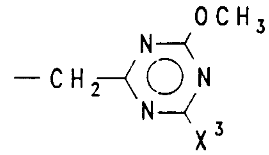 ,

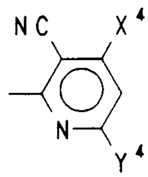 oder 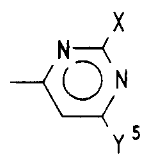

| | |
|---|---|
| X,Y | unabhängig voneinander H, Halogen, $(C_1\text{-}C_3)$Alkyl, $(C_1\text{-}C_3)$Alkoxy oder $(C_1\text{-}C_3)$Alkylthio, wobei die vorgenannten alkylhaltigen Reste unsubstituiert oder ein- oder mehrfach durch Halogen oder ein-oder zweifach durch $(C_1\text{-}C_3)$Alkoxy oder $(C_1\text{-}C_3)$Alkylthio substituiert sind, oder einen Rest der Formel $NR^5R^6$, $(C_3\text{-}C_6)$Cycloalkyl, $(C_2\text{-}C_4)$Alkenyl, $(C_2\text{-}C_4)$-Alkinyl, $(C_3\text{-}C_4)$-Alkenyloxy oder $(C_3\text{-}C_4)$Alkinyloxy, |
| $R^5$,$R^6$ | unabhängig voneinander H, $(C_1\text{-}C_3)$Alkyl oder $(C_3\text{-}C_4)$Alkenyl, |
| W | O oder S, |
| Z | CH oder N, |
| $X^1$ | $CH_3$, $OCH_3$, $OC_2H_5$ oder $OCF_2H$, |
| $Y^1$ | -O- oder $-CH_2-$, |
| $X^2$ | $CH_3$, $C_2H_5$ oder $CH_2CF_3$, |
| $Y^2$ | $OCH_3$, $OC_2H_5$, $SCH_3$, $SC_2H_5$, $CH_3$ oder $C_2H_5$, |
| $X^3$ | $CH_3$ oder $OCH_3$, |
| $Y^3$ | H oder $CH_3$, |
| $X^4$ | $CH_3$, $OCH_3$, $OC_2H_5$, $CH_2OCH_3$ oder Cl, |
| $Y^4$ | $CH_3$, $OCH_3$, $OC_2H_5$ oder Cl, |
| $Y^5$ | $CH_3$, $C_2H_5$, $OCH_3$ oder Cl bedeuten. |

**2.** Verbindungen oder deren Salze nach Anspruch 1, dadurch gekennzeichnet, daß

$R^1$ H, $(C_1\text{-}C_4)$Alkyl, das unsubstituiert oder durch 1 bis 3 Reste aus der Gruppe Halogen oder durch $(C_1\text{-}C_2)$Alkoxy oder $(C_3\text{-}C_5)$Cycloalkyl substituiert ist, bedeutet oder

$R^2$ H, $(C_1\text{-}C_4)$Alkyl, das unsubstituiert oder durch ein oder mehrere Halogenatome oder durch $(C_1\text{-}C_2)$Alkoxy substituiert ist, oder $(C_2\text{-}C_4)$Alkenyl, $(C_2\text{-}C_3)$Alkinyl, $(C_3\text{-}C_5)$Cycloalkyl, $(C_1\text{-}C_4)$-Alkoxy, das unsubstituiert oder durch ein oder mehrere Halogenatome oder durch Phenyl substituiert ist, oder einen Rest der Formel $NR^aR^b$, worin $R^a$ und $R^b$ unabhängig voneinander H oder $(C_1\text{-}C_4)$Alkyl sind, bedeutet oder

$R^3$ $(C_1\text{-}C_4)$Alkyl, $(C_1\text{-}C_3)$Haloalkyl, Halogen, $(C_1\text{-}C_3)$Alkoxy oder Nitro bedeutet oder

A ein Rest der Formel

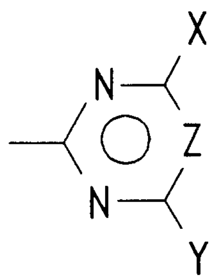

ist, worin einer der Reste X und Y $(C_1-C_3)$Alkyl, $(C_1-C_3)$Alkoxy, $(C_1-C_3)$Haloalkyl, $(C_1-C_3)$Haloalkoxy oder $[(C_1-C_2)$Alkoxy$]-(C_1-C_2)$alkyl und der andere Rest Y bzw. X $(C_1-C_3)$Alkyl, $(C_1-C_3)$Alkoxy, $(C_1-C_3)$-Alkylthio, wobei jeder der letztgenannten 3 Reste unsubstituiert oder ein- oder mehrfach durch Halogen oder ein- oder zweifach durch $(C_1-C_3)$Alkoxy oder $(C_1-C_3)$Alkylthio substituiert ist, oder Halogen oder ein Rest der Formel $NR^5R^6$, worin $R^5$ und $R^6$ unabhängig voneinander H, $(C_1-C_3)$Alkyl oder $(C_3-C_4)$-Alkenyl sind, oder $(C_3-C_6)$Cycloalkyl, $(C_2-C_4)$Alkenyl, $(C_2-C_4)$Alkinyl, $(C_3-C_4)$Alkenyloxy oder $(C_3-C_4)$-Alkinyloxy bedeuten.

**3.** Verbindungen oder deren Salze nach Anspruch 1 oder 2, dadurch gekennzeichnet, daß
$R^1$ H, $(C_1-C_4)$Alkyl, $(C_1-C_4)$Haloalkyl oder $[(C_1-C_2)$Alkoxy$]-(C_1-C_2)$alkyl,
$R^2$ H, $(C_1-C_4)$Alkyl, Halo-$(C_1-C_4)$alkyl, $(C_2-C_4)$Alkenyl, $(C_2-C_4)$Alkinyl, $(C_1-C_4)$Alkoxy, $[(C_1-C_2)$-Alkoxy$]-(C_1-C_2)$alkyl, $(C_3-C_5)$Cycloalkyl oder einen Rest der Formel $NR^aR^b$, worin $R^a$ und $R^b$ unabhängig voneinander H oder $(C_1-C_4)$Alkyl sind,
$R^3$ $(C_1-C_4)$Alkyl, Halogen, Nitro oder $(C_1-C_4)$Alkoxy und
n 0 oder 1 bedeuten.

**4.** Verbindungen oder deren Salze nach einem der Ansprüche 1 bis 3, dadurch gekennzeichnet, daß einer der Reste X oder Y $(C_1-C_2)$Alkyl, $(C_1-C_2)$Alkoxy oder $OCF_2H$ und der andere Rest Y bzw. X $(C_1-C_2)$Alkyl, $(C_1-C_2)$Alkoxy, Halogen, $OCF_2H$, $OCH_2CF_3$ oder $CF_3$ bedeuten.

**5.** Verfahren zur Herstellung der Verbindungen der Formel (I) oder deren Salze, wie sie in Anspruch 1 definiert sind, dadurch gekennzeichnet, daß man
a) eine Verbindung der Formel (II),

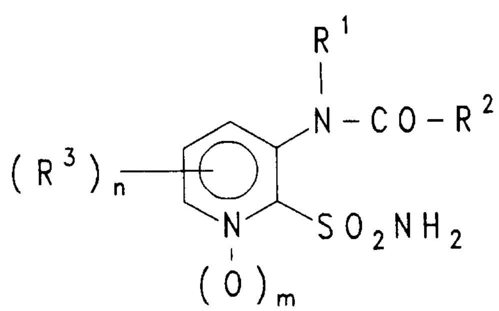

(II)

mit einem heterocyclischen Carbamat der Formel (III),

$R^*$-O-CO-$NR^4$-A (III)

worin $R^*$ Phenyl oder substituiertes Phenyl oder $(C_1-C_4)$Alkyl bedeutet, umsetzt oder
b) ein Pyridylsulfonylcarbamat der Formel (IV),

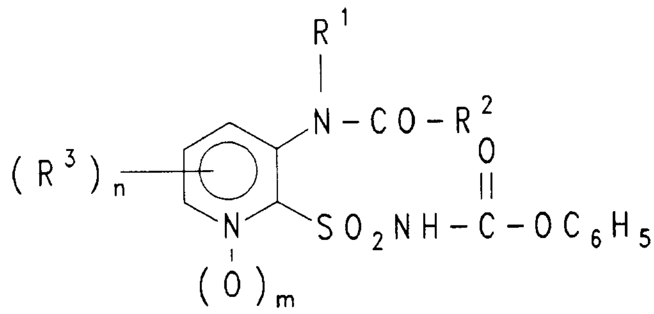

(IV)

mit einem Aminoheterocyclus der Formel (V)

$H\text{-}NR^4\text{-}A$ (V)

umsetzt oder

c) ein Sulfonylisocyanat der Formel (VI)

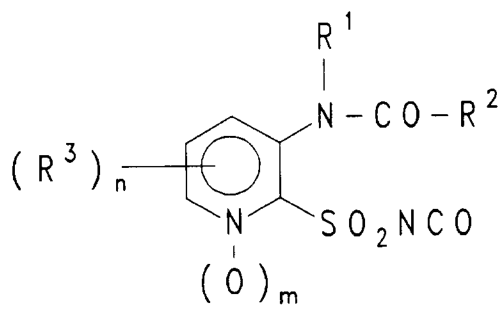

(VI)

mit einem Aminoheterocyclus der Formel (V)

$H\text{-}NR^4\text{-}A$ (V)

umsetzt oder

d) in einer Eintopfreaktion zunächst einen Aminoheterocyclus der Formel $H\text{-}NR^4\text{-}A$ (V) in Gegenwart einer Base mit Phosgen umsetzt und das gebildete Intermediat mit einem Pyridinsulfonamid der Formel (II) umsetzt,

wobei in den Formeln (II)-(VI) $R^1$, $R^2$, $R^3$, $R^4$, A, m und n wie in Formel I definiert sind.

**6.** Herbizides oder pflanzenwachstumsregulierende Mittel, dadurch gekennzeichnet, daß es mindestens eine Verbindung der Formel (I) oder deren Salz nach einem der Ansprüche 1 bis 4 und im Pflanzenschutz übliche Formulierungshilfsmittel enthält.

**7.** Verfahren zur Bekämpfung von Schadpflanzen oder zur Wachstumsregulierung von Pflanzen, dadurch gekennzeichnet, daß man eine wirksame Menge von mindestens einer Verbindung der Formel (I) oder deren Salzen nach einem der Ansprüche 1 bis 4 auf die Schadpflanzen bzw. Pflanzen, deren Pflanzensamen oder die Fläche, auf die Pflanzen wachsen, appliziert.

**8.** Verwendung der Verbindungen der Formel (I) oder deren Salze nach einem der Ansprüche 1 bis 4 als Herbizide oder Pflanzenwachstumsregulatoren.

**9.** Verbindungen der Formel (II),

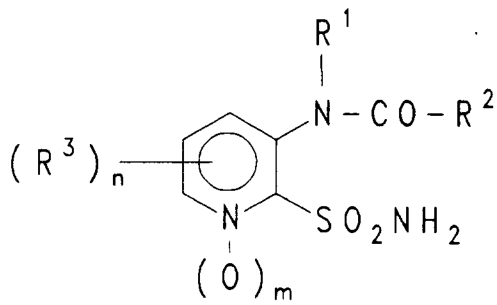

(II)

worin $R^1$, $R^2$, $R^3$, m und n wie in Formel I nach einem der Ansprüche 1 bis 4 definiert sind.

**10.** Verfahren zur Herstellung der Verbindungen der Formel (II) nach Anspruch 9, dadurch gekennzeichnet, daß man Verbindungen der Formel (VII),

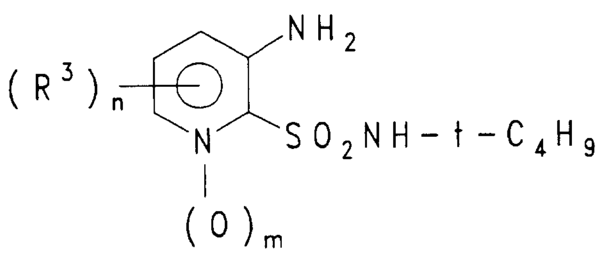

(VII)

worin $R^3$, m und n wie in Formel (II) definiert sind, mit Carbonsäurehalogeniden der Formel Hal-CO-$R^2$ (Hal = Halogen) oder symmetrischen oder gemischten Carbonsäureanhydriden der Formel $R^2$-CO-O-CO-$R^+$ , worin $R^+$ analog $R^2$ definiert ist oder ein anderer aliphatischer oder aromatischer Rest ist, umsetzt und anschließend das erhaltene, mit der t-Butylgruppe geschützte 3-(N-Acylamino)-pyridin-2-sulfamid

a) mit einer starken Säure zum freien 3-(N-Acylamino)-pyridin-2-sulfamid der Formel (II), in der $R^1$ = H ist, umsetzt oder

b) zur N-Alkylverbindung reduziert, die N-Alkylverbindung mit einer Verbindung der Formel Hal-CO-$R^2$ oder $R^2$-CO-O-CO-$R^+$ N-acyliert und die t-Butyl-Schutzgruppe mit einer starken Säure abspaltet, wobei das freien 3-(N-Acylamino)-pyridin-2-sulfamid der Formel (II), in der $R^1$ ungleich Wasserstoff ist, erhalten wird.

Europäisches Patentamt

# EUROPÄISCHER RECHERCHENBERICHT

Nummer der Anmeldung

EP 93 10 1723

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| L<br><br><br><br>A,P | CHEMICAL ABSTRACTS, vol. 118, no. 15, 12. April 1993, Columbus, Ohio, US; abstract no. 147571a, Seite 844 ;<br>& JP-A-04 253 974 (ISHIHARA SANGYO KAISHA, LTD.) 9. September 1992<br>--- | 1,2,5-9 | C07D401/12<br>C07D491/04<br>C07D213/75<br>A01N47/36<br>//(C07D401/12, 239:00,213:00)<br>(C07D401/12, 251:00,213:00)<br>(C07D401/12, 249:00,213:00)<br>(C07D491/04, 307:00,239:00)<br>(C07D491/04, 311:00,239:00) |
| A,D | WO-A-9 110 660 (HOECHST AG)<br>* Ansprüche 1-8; Verbindungen Nr. 354-410, 439-698, 921-938, 940, 941, 955-967 *<br>--- | 1,2,5-9 | |
| A | EP-A-0 459 949 (CIBA-GEIGY AG)<br>* Ansprüche 1, 15-20, 22; Tabelle 1 *<br>--- | 1,2,5-9 | |
| A | EP-A-0 451 468 (ISHIHARA SANGYO KAISHA, LTD.)<br>* Ansprüche 1, 2, 5, 7-10, 12, 13; Tabelle 2 *<br>--- | 1,2,5-9 | |
| A,D | EP-A-0 237 292 (E.I. DU PONT DE NEMOURS AND CO.)<br>* Ansprüche 1, 12-16; Tabelle 1 *<br>----- | 1,2,5-8 | **RECHERCHIERTE SACHGEBIETE (Int. Cl.5)**<br>C07D<br>A01N |

Der vorliegende Recherchenbericht wurde für alle Patentansprüche erstellt

| Recherchenort | Abschlußdatum der Recherche | Prüfer |
|---|---|---|
| BERLIN | 24 MAI 1993 | HASS C. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)